# EUROPEAN PATENT SPECIFICATION

(11) **EP 3 448 354 B1**
(45) Date of publication and mention of the grant of the patent: **16.07.2025**
(21) Application number: 17714512.5
(22) Date of filing: 16.03.2017
(51) Int. Cl.: A61K 8/41, A61K 8/46, A61Q 5/02, A61Q 9/02, A61K 8/81, A61Q 19/10, A61K 8/04

(54) **POST-FOAMING MILD CLEANSING COMPOSITION**
NACHSCHÄUMENDE MILDE REINIGUNGSZUSAMMENSETZUNG
COMPOSITION DE NETTOYAGE DOUCE POST-MOUSSAGE

(30) Priority: 26.04.2016 GB 201607198
(43) Date of publication of application: 06.03.2019
(73) Proprietor: PZ Cussons (International) Limited, Manchester M22 5TG (GB)
(72) Inventor: HEFFERNAN, Paul, Northwich Cheshire CW8 2HQ (GB); SHAW, Phillip, Bowdon Cheshire WA14 2TZ (GB); WU, Meiki, Manchester Lancashire M43 6GG (GB); DAVIES, Craig, Stoke-on-Trent Staffordshire ST7 2XG (GB)
(74) Representative: Wilson Gunn
(86) International application number: PCT/GB2017/050725
(87) International publication number: WO 2017/187124

(56) References cited:
- WO-A1-95/13349
- CA-A1- 2 352 288
- US-A1- 2006 147 406
- US-A1- 2012 046 210

## Description

The present invention relates to post-foaming personal cleansing compositions in gel or cream form, which are particularly mild, can contain high levels of emollient skin-care ingredients and produce copious amounts of creamy foam in use. The use of the herein described novel structuring system enables stable post-foaming gels or creams to be made while combining unusually low surfactant levels with high levels of emollient oils. The present invention also relates to a method of manufacturing the personal cleansing composition, and use of the composition as a personal cleansing composition.

The present invention provides an effective solution to the dual consumer need for highly skin caring cleansing formulations with excellent foaming characteristics, as conventional formulations with a combination of low surfactant levels with high levels of emollient oils have suffered from poor in-use foaming performance, and so are rejected by consumers.

### Background to the invention

Post-foaming formulations that are suitable for personal cleansing applications are known in the art. However, these formulations typically require high levels of anionic surfactant to produce a stable gel structure. As a result, there is only a limited amount of flexibility for improving mildness by using lower levels of surfactants or utilising milder types of surfactants, or for the incorporation of significant levels of emollient oils, as these tend to destabilise the gel structure.

Personal cleansing formulations with high oil loading that do not use post-foaming technology are described in the art. Unstructured personal cleansing liquids are unsuitable for holding high levels of insoluble/partially soluble oils as they will be unstable due to coalescence and 'creaming' of the oil towards the surface due to having a lower density than the aqueous medium. This can be solved by using an appropriate structuring system, using either 'external' structuring agents or structured surfactant systems.

This problem is well understood in the art, and various solutions have previously been described and commercialised. Essentially, the solutions fall into two broad camps:
A) Use 'external' structurants within the aqueous phase of the product. These external structurants include natural gums such as xanthan and guar derivatives, and synthetic polymers such as carbomers and acrylate derivatives and clays (natural or synthetic). These materials are sometimes used in combination; or
B) Use structured surfactant systems. These rely on aggregation structures formed by the surfactants, typically lamellar in nature, which can either be in the form of parallel sheets or multi-lamellar vesicles known as spherulites. Such systems require precise combinations of particular surfactants with the right physical shape, and also sometimes require an inducer which may be a simple electrolyte such as sodium chloride or a fatty acid or fatty alcohol.

Examples of prior compositions which fall under type (A):
US 6,001,344 describes a novel structuring system comprising both xanthan gum and cross-linked polyacrylic acid polymer which is said to provide enhanced stability for large size benefit agent droplets in an amphoteric/anionic surfactant system relative to either structurant alone.
US 6,846,785 describes a liquid soap having vitamin containing microcapsules including a base have an anionic surfactant and a chelating agent, a cross-linked acrylic polymer suspending agent, and multiple vitamin-containing microcapsules uniformly suspended in the liquid soap. The liquid soap is formulated at an elevated temperature which is maintained throughout the formulation process.

Examples of prior compositions which fall under type (B):
US 7,488,707 describes an aqueous structured surfactant system containing water, one or more anionic surfactants, and one or more alkoxylated nonionic surfactants, which is said to exhibit shear-thinning viscosity, be capable of suspending water insoluble or partially water soluble components, and be mild to the eyes and skin.
US 2008/0233061 describes a structured surfactant composition, comprising i) from greater than 0 to 15 parts by weight of an alkyl ether sulfate surfactant, ii) from greater than 0 to 15 parts by weight of an alkyl sulfate surfactant, iii) from greater than 0 to 8 parts by weight of an alkanolamide surfactant, iv) from 0 to 10 parts by weight of an amphoteric surfactant, providing that the total amount of components (i), (ii), (iii) and (iv) is greater than or equal to 5 parts by weight, v) an amount of electrolyte effective in combination with (i), (ii), (iii) and (iv), providing a structured surfactant composition having an opaque visual appearance and exhibiting a yield strength of greater than 0 Pascals, and (vi) water.
US 5,556,628 describes storage-stable, pseudoplastic free-flowing cosmetic compositions/formulations, for example shampoos, shower and exfoliating gels and hair lotions, which comprise a stable and homogeneous suspension, in water, of water-insoluble particulates, and which further comprise at least one anionic surfactant, at least one nonionic or amphoteric surfactant and at least one electrolyte, these surfactants being present in such amounts as to impart pseudoplasticity thereto with a yield point of at least 0.2 Pa and constituting spherulites suspended within a lamellar phase.
US 5,952,286 describes lamellar phase compositions comprising defined surfactant systems and a structurant selected from liquid fatty acids, liquid alcohols and derivatives thereof, where the structurant is responsible for inducing the lamellar phase.
WO 2009/063250 describes an aqueous shear-thinning personal care composition comprising: a) at least 3% of a sulfonate surfactant of general formula (I): 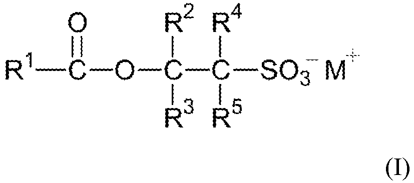 b) at least 3% of an amphoteric or zwitterionic surfactant; c) at least 10% of water; and d) at least 0.5% of an additional component in the form of particles or droplets suspended in the composition.

Example of prior compositions which are based on elements of both approaches:
WO 2011/057909 describes a mild, substantially isotropic skin cleansing solution able to suspend insoluble components and produce lather. The cleanser is formulated with synthetic anionic surfactants and a specific ratio of carboxylic acid(s) to hydrophobically modified cross-linked acrylate copolymer(s). The carboxylic acid and acrylate polymer combination were said to provide a synergistic effect on zero shear viscosity at 25°C in a specific pH and copolymer/acid concentration ratio range.

While these approaches can be effective at providing the necessary rheology to suspend insoluble/partially soluble matter, they suffer from a number of disadvantages.

Products structured with natural gums, such as those described in US 2004/0121925, have undesirable consumer rheology, appearing thick at rest but unacceptably runny in use, sometimes perceived as inconsistent or 'gloopy'.

Products that use synthetic polymers are considered to have much better flow behaviour; however, they are typically expensive due to the high cost and inclusion level of such materials. Some polymers, such as carbomers, are also very sensitive to electrolytes and surfactants, which reduces their effectiveness.

Compositions based on structured surfactant systems typically require specialised blends of surfactants which are need to be added at a high level of inclusion, and have only limited flexibility in terms of their ability to include other types of surfactants or benefit agents, as these may affect the overall 'structurisation'. The desired product texture usually requires the incorporation of high levels of oil (typically 15-50%). While in some cases this may be desirable, it significantly increases the formulation or finished product costs. It would be more desirable to have the flexibility and freedom to vary the oil level from zero to 50%, without the prohibitive cost implications.

Further, in some cases, hot processes are required to melt solid ingredients such as fatty acids; cold mixing is preferable as this shortens production time as no heating and cooling steps are required, and also reduces energy requirements, and thus reduces the overall cost of production.

None of the above examples, however, describe the use of their base formulations into a post foaming delivery execution. The key disadvantage of both routes (A) and (B) is the foaming performance is generally poor, especially if high levels of oil are incorporated. They therefore have limited consumer appeal.

Prior art that *does* disclose the use of post foaming systems, on the other hand, does not teach the incorporation of oils at significant levels, and the benefits that this confers:
US4726944 by Osipow et al describes the use of amine oxide surfactants in post foaming gel systems. However, they are used in combination with an aqueous soap solution and free fatty acids, and as such will be harsh on the skin, and will rely upon the gel structure being generated by the soap and fatty acid components of the mixture combined with a water soluble gum. It also proposes that the formulation be packaged in a non-aerosol format; however, in reality this is impractical due to the pressures generated by the volatile hydrocarbon foaming agent at elevated temperatures.

GB2236760 by Mann & Garien describes the use of *in situ* neutralised polymers in the formation of a post foaming gel. However, this invention is focused specifically on the delivery of a shaving preparation and uses a specific class of polymer, and does not rely upon the neutralisation of the polymer by a surface active agent within the formula to develop its structure.

WO2004004678 by Kuether & Rohde describes the use of starch derived polymers in combination with an anionic surfactant system.

US5962396 by Pollack & Gomes describes the use of an amine oxide in a post-foaming gel cleaning system; however, this uses a sulfonated fatty acid base combined with potassium hydroxide as a neutralising agent in order to form a soap. This then is combined with a synthetic sultaine surfactant.

WO 95/13349 discloses a stable, self-foaming cleansing composition, which has a self-foaming agent, an active suspensate and low levels of a surface active agent, but does not disclose a composition comprising a HASE polymer or an amine oxide.

US 2006/147406 discloses viscous products formulated from two low viscosity component formulations, wherein the post-foaming compositions contain water, sodium lauryl sulfate, oleamine oxide, acrylate/steareth-20 itaconate copolymer, and isopentane, have low viscosity and are self-thickening.

There is therefore a need for a personal cleansing composition which is able to overcome one or more of the above problems, and is able to address the dual challenges of delivering excellent foaming performance with a high degree of mildness to skin by utilising post-foaming technology.

Therefore, in accordance with the present invention there is provided a post-foaming composition comprising:
a) water;
b) one or more anionic surfactants in an amount of about 2 to about 20% by weight of the composition;
c) one or more amine oxide surfactants in an amount of about 1 to about 10% by weight of the composition;
d) a hydrophobically modified alkali-swellable acrylic emulsion (HASE) polymer in an amount of about 0.5 to about 3% polymer solids by weight of the composition;
e) a water-insoluble oil in an amount of about 1% to about 50% by weight of the composition, wherein the solubility of the substantially water-insoluble oil in water at 20°C is less than 50% by weight of a water/oil mixture; and
f) a foaming agent comprising a non-polar saturated aliphatic hydrocarbon having from 3 to 6 carbon atoms.

The concept of 'alkali-swellable' polymers is well known in the polymer technical field, and as such will be familiar to, and understood by, a person skilled in the art. Such HASE polymers are also commercially available, for example under the ACULYN^{®} trade name from Dow Chemicals, or under the Rheovis^{®} and Latekoll^{®} trade names from BASF.

In their commercially available forms, the polymers (which are acidic in their nature) are coiled up tightly and dispersed in an aqueous medium, essentially as insoluble particles. In the presence of an alkali, the acidic polymer is neutralized, and expands due to negative charge repulsion. The particles of polymer are therefore said to swell, with water filling up the spaces. Not all synthetic anionic polymers swell in the presence of alkalis, for example the type used as opacifying agents (e.g. styrene/acrylates copolymer) are designed to remain in a compact state.

When HASE polymers/rheology modifiers are neutralized, they become anionically charged and water-soluble. When HASE polymers dissolve, they swell due to charge-to-charge repulsion and thicken instantly. As in the case of cross-linked alkali-swellable emulsion (ASE) polymers, structure is built as the critical concentration is exceeded, but in the case of the HASE rheology modifiers, it is by polymer chain entanglement, in conjunction with association of the extended hydrophobic groups on the polymer backbone with each other and other hydrophobic groups in a formulation.

The invention uses a combination of an anionic surfactant combined with an amine oxide surfactant, a substantially water-insoluble oil, and a HASE polymer. The resulting combination is then blended with a post-foaming agent and packaged into a bi-compartmental or other suitable aerosol system.
The combination of anionic surfactant and amine oxide surfactant provides a relatively mild and low cost cleansing mixture. It is milder than typical high anionic (either synthetic or soap based) systems and delivers acceptable performance at a cost that is typically less than equivalently performing systems based upon synthetic surfactants. A key driver of this benefit is that relatively low levels of surfactant are required to create a consumer acceptable product.

The inventors have found that resulting anionic-amine oxide surfactant mixture interacts strongly with the HASE polymer, enabling the use of lower polymer and surfactant inclusion levels than those which would normally be required to provide a sufficiently high viscosity at rest necessary for stabilisation of dispersed materials such as oil droplets.

The addition of the oil further improves the skin care qualities of the composition. The oil is emulsified into the base gel with simple mixing, stabilised by the HASE polymer due to hydrophobic interactions with the pendant groups. The presence of the oil can confer a high degree of opacity and a cream-like texture to the composition. Such attributes are highly desirable in meeting consumer expectations for a product designed to care for skin. Alternatively, if desired, transparent oil-containing gels may also be formulated using appropriate selection of surfactants and oils. It is commonly understood in the art that incorporation of oil into a cleansing composition is technically beneficial, as a proportion of said oils may deposit onto the skin during washing thus compensating for skin lipids that are removed by surfactants. Furthermore, while not wishing to be bound by any particular theory, it is believed that the presence of oils reduces the capacity of surfactants in the formulation to interact with skin lipids as their hydrophobic 'tails' are already 'engaged' with the oil droplets in the formulation.

When a suitable post-foaming agent is mixed into the composition it becomes uniformly incorporated with little or no change in appearance. This can be transferred into a suitable aerosol container (ideally bi-compartmental) under pressure. When dispensed from the container the product emerges with an appearance of a gel/cream, and when applied to wet skin or hair, the warmth and moderate shearing forces results in the post-foaming agent emerging as a gas which transforms the gel/cream into copious amounts of rich, creamy foam.

The present invention addresses the dual challenges of delivering excellent foaming performance with a high degree of mildness to skin by utilising post-foaming technology. The solution the inventors have developed inherently produces a significantly greater foam volume than conventional and commercially available products. The present invention therefore has the potential to compensate for an inherently poor foaming base system, which creates the opportunity to use high emollient levels, low surfactant levels and surfactant types that are mild but not particularly good at foaming when used in conventional (i.e. non post-foaming) products.

The problem in achieving this in a post-foaming format is that the known prior art in the field of post-foaming cleansing compositions would be unstable if pushed in this direction because formation of the gel structure, and its ongoing stability, requires a fine balance of particular surfactant levels and post-foaming agent(s), typically requiring a relatively high level of surfactants that is counter-productive for skin care as they tend to remove skin lipids. The present invention addresses this challenge by utilising a novel structuring system that is surprisingly robust, enables the use of low surfactant levels and tolerates high levels of emollient oils which in principle can deliver more lipids to the skin than the cleansing process removes.

The composition is packaged in to an aerosol container or any such container that can dispense a post-foaming gel. This may be a bi-compartmental aerosol container. A bi-compartmental aerosol container than can be used to contain the composition of the invention may take the form of a bag in can, bag on valve, piston, elasticated bladder or other barrier system. Application of an external pressure to the formulation-containing compartment enables the formulation to be dispensed on opening a valve, actuated by the consumer during product usage.

The pressure must be sufficient to prevent the post-foaming agent transforming to a gaseous state inside the formulation compartment, even at elevated temperature. This may be applied either by mechanical means (such as an elasticated bladder system) or via an 'external' propellant that resides in the space between the formulation compartment and the outer container. In the case of the latter the external propellant gas may be either a liquefied or compressed gas, such as a C1-C4 hydrocarbon, a fluorocarbon, nitrous oxide, compressed air or nitrogen.

A further advantage of this novel approach is that it is can use very mild anionic surfactants such as sulfosuccinates, sarcosinates, glutamates etc as primary surfactants. Traditionally, they are used only as co-surfactants due to cost and performance limitations. The ability for this system to structure up effectively and produce a good level of foam at low active levels makes the use of these mild anionic surfactants a realistic proposition where the aim is to deliver a higher degree of mildness. The resulting formulations have a desirably thick but shear-thinning gel texture. It is flexible in its ability to allow the stable incorporation of oils up to at least 50%. It is also easy to manufacture, has good lathering characteristics and has been found to leave the skin feeling soft and smooth.

### HASE Polymer Constituent:

HASE polymers or thickeners are well known in the art, and are the associative analogue to alkali-swellable emulsion ASE synthetic thickeners which are produced commercially by emulsion polymerisation. Like the ASE thickeners, HASE polymers have 'backbones' made from methacrylic acid and various acrylate/methacrylate alkyl ester monomers. However, unlike typical ASE types, HASE polymers contain hydrophobic "pendants" which are commonly attached via polyethylene oxide chains. Thickening is achieved by 2 mechanisms: firstly, upon addition of an alkaline neutralising base (for example an alkali metal hydroxide), the -COOH moieties along the backbone become ionised to -COO⁻ carboxyl groups which cause the polymer chain to expand by electrostatic repulsion and become soluble in water, changing from a milky low viscosity dispersion to a transparent gel. Secondly, the hydrophobic pendant groups are able to build associations with each other and also with surfactants, oil droplets and any other dispersed particulates. This creates a network structure that is accompanied by significant viscosity build.

Suitable HASE polymers for use in the present invention are anionic in nature upon neutralisation. They are typically supplied in the form of an aqueous liquid emulsion (which has an un-neutralised, acidic pH) form with hydrophobic appendages which make them 'associative' - *i.e.* they can associate with other formulation ingredients, such as surfactants, oils or particles.

Suitable HASE polymers include, but are not limited to, those commercially available from Rohm & Haas under the Aculyn trade name, such as Aculyn 22 (acrylates/steareth-20 methacrylate copolymer), Aculyn 28 (acrylates/beheneth-25 methacrylate copolymer) and Aculyn 88 (acrylates/steareth-20 methacrylate crosspolymer). Other options include Synthalen W2000 from 3V (acrylates/palmeth-25 acrylate copolymer), and Novethix L-10 (acrylates/beheneth-25 methacrylate copolymer) from Lubrizol.

The HASE polymer is present in an amount of about 0.5 to about 3% by weight (as polymer solids) of the total composition, typically from about 0.5 to about 2% by weight, more typically from about 1.0 to about 1.5% by weight.

### Anionic Surfactant Constituent:

Suitable anionic surfactants for use in the composition of the invention include, but are not limited to, alkyl sulfates (*e.g*. sodium lauryl sulfate or sodium cocosulfate); ethoxylated alkyl sulfates (*e.g*. sodium laureth sulfate or sodium trideceth sulfate); alpha olefin sulfonates; alkyl alkoxy carboxylates; acyl isethionates (*e.g*. sodium cocoyl isethionate); acyl sarcosinates (*e.g.* sodium cocoyl sarcosinate); sulfosuccinates (*e.g.* disodium laureth sulfosuccinate); or acyl glutamates such as sodium cocoyl glutamate. Alternative counter ions may be used in place of sodium, such as potassium, ammonium, triethanolammonium (TEA), or monoisopropylammonium (MIPA). According to one embodiment, the anionic surfactant includes, or is, sodium laureth sulfate.

The one or more anionic surfactants are present in an amount of about 0.5 to about 20% by weight of the composition; typically from about 1 to about 10% by weight, more typically from about 2 to about 6% by weight.

### Amine Oxide Constituent:

Suitable amine oxide surfactants for use in the composition of the invention include, but are not limited to, alkamine oxides containing at least one long hydrocarbon chain containing at least eight carbon atoms.

One such class of amine oxides is the alkyl di(lower alkyl) amine oxides, wherein the alkyl group therein contains from 8 to 22, and preferably about 10 to about 16, carbon atoms, and can be straight or branched chain, saturated or unsaturated. The lower alkyl groups in the alkyl di(lower alkyl) amine oxides contain from 1 to 7 carbon atoms, and typically are methyl, ethyl, propyl or butyl, more typically methyl or ethyl. Specific examples include, but are not limited to, lauryl dimethyl amine oxide, myristyl dimethyl amine oxide, dimethyl cocoamine oxide, dimethyl (hydrogenated tallow)amine oxide, myristyl/palmityl dimethyl amine oxide, myristyl/lauryl dimethyl amine oxide, cetyl dimethyl amine oxide, stearyl dimethyl amine oxide, and myristyl/cetyl dimethyl amine oxide.

Another class of useful amine oxides for use in the present invention includes alkyl di(hydroxy lower alkyl)amine oxides in which the alkyl group contains from 8 to 22, and preferably about 10 to about 16 carbon atoms, and can be straight or branched chain, saturated or unsaturated. The lower alkyl groups in the alkyl di(lower alkyl) amine oxides contain from 1 to 7 carbon atoms, and typically are methyl, ethyl, propyl or butyl, more typically methyl or ethyl. Specific examples, include, but are not limited to, bis(2-hydroxyethyl)cocoamine oxide, and bis(2-hydroxyethyl)stearylamine oxide.

Additional useful amine oxides for use in the present invention are termed alkamidopropyl di(lower alkyl)amine oxides in which the alkyl group contains from 8 to 22, and preferably about 10 to about 16 carbon atoms, and can be straight or branched chain, saturated or unsaturated. The lower alkyl groups in the alkyl di(lower alkyl) amine oxides contain from 1 to 7 carbon atoms, and typically are methyl, ethyl or propyl, more typically methyl. Non-limiting examples thereof are cocoamidopropyl dimethyl amine oxide and lauramidopropyl dimethyl amine oxide.

The above classes of alkamine oxide surfactants contain one or more C8-C22 alkyl groups selected from, for example, octyl, decyl, undecyl, lauryl, tridecyl, myristyl, cetyl, stearyl, isostearyl, oleyl, and mixtures thereof. Non-limiting examples of amine oxide surfactants include, but are not limited to, decyl dimethylamine oxide, lauryl dimethylamine oxide, stearyl dimethylamine oxide, oleyl dimethylamine oxide, coco dihydroxyethylamine oxide, cetyl N,N-dihydroxyethylamine oxide, oleyl N,N-dihydroxyethylamine oxide, cocamine oxide, cocamidopropylamine oxide, lauramidopropylamine oxide, oleamine oxide, oleamidopropylamine oxide, wheat germamidopropylamine oxide, isostearamido-propylamine oxide, stearamine oxide, stearamido-propylamine oxide, decylamine oxide, dihydroxyethyl C8-C18 alkoxypropylamine oxide, myristamidopropylamine oxide, myristamine oxide, palmitamidopropylamine oxide, palmitamine oxide, undecylenamidopropylamine oxide, and mixtures of any two or more thereof. Preferred alkamine oxide surfactants are the alkyl di(lower alkyl)amine oxides in which the alkyl group contains about 12 to about 16 carbon atoms, including lauramine oxide, myristamine oxide, cocamine oxide, cetamine oxide, and mixtures of any two or more thereof. According to one embodiment of the invention, the amine oxide surfactant comprises, or is, lauramine oxide.

The one or more amine oxide surfactants are present in an amount of about 1 to about 10% by weight of the composition; typically from about 0.5 to about 8.0% by weight, more typically from about 1.0 to about 5.0% by weight, more typically from about 1.5 to about 3.0% by weight.

### Foaming Agent Constituent

Typically, the foaming agent comprises one or more compounds selected from iso-pentane, n-pentane, neo-pentane, iso-butane or n-butane. Typically, the foaming agent constitutes from about 2% to about 20%, and preferably between about 5% to about 15%, by weight of the composition.

By 'post-foaming' in the context of the invention, it is meant that the composition contains a volatile foaming agent that transforms from a liquid or gelled state (by being dissolved, emulsified or otherwise entrapped in the formulation matrix) to a gaseous state *after* dispensation from its retail packaging, and during intended usage, to generate foam *internally* within the applied formulation, by virtue of this change of physical state of said agent. Unlike conventional cleansing products such formulations are therefore not reliant on entrainment of atmospheric air by mechanical action to generate foam.

### Emollient Oil Constituent

Suitable oils include those of vegetable origin, plant derived butters, mineral oils (*e.g.* petroleum derived or otherwise extracted and refined from rock/mineral deposits found within the Earth's crust), animal derived (*e.g*. squalene), or synthetic such as silicones. Chemically the oils may be long-chain hydrocarbons, tri-acyl glycerides, man-made esters formed from reaction of carboxylic acids with alcohols, or silicones. As used herein, the definition of water-insoluble oils does not include C₃-C₆ aliphatic hydrocarbons which are used as the foaming agent.

Vegetable triglycerides, mineral oil, petrolatum, hydrocarbons (excluding C₃-C₆ aliphatic hydrocarbons, as indicated above), silicones, plant derived butters, esters, essential oils, or compounded fragrance oils may be used.

Oils that are of vegetable origin for use in the invention include sunflower oil, corn oil, rapeseed oil, ground nut oil, almond oil, jojoba oil, petrolatum, lanolin, coconut oil, palm oil, palm kernel oil and plant butters, etc.

The emollient oil constituent is present in an amount of about 1 to about 50% by weight of the composition; typically from about 1.5 to about 30% by weight, more typically from about 2 to about 20% by weight.

According to another embodiment of the invention, the composition may further contain one or more of the following components:
Natural or synthetic scrub particles (*e.g*. polyethylene, silica, rice bran, loofah, pumice, diatomaceous earth, pearlite, ground fruit or nut 'stones' or shells), visual effect agents (*e.g.* wax particles, polymeric opacifiers, inorganic 'shimmers'), humectants (*e.g.* glycerol, sorbitol, glycols, urea, amino acids, lactates), cationic conditioning agents (such as cationic guar or cassia gum derivatives, Polyquaternium-7, Polyquaternium-39, Polyquaternium-10, etc) preservatives, colourants, chelating agents and pH adjusters (acids or bases). The formulation may optionally include a clay based rheology modifier such as a bentonite, hectorite, montmorillonite or synthetic clay such as Laponite (Rockwood Products) which is available in various grades.

The various components of the composition of the invention are added in their respective amounts as desired; the balance of the composition to 100% is water.

The composition of the invention may further contain one or more ingredients selected from emollients, moisturizers, conditioners, skin conditioners, hair conditioners, vitamins, pro-vitamins, antioxidants, free-radical scavengers, abrasives, dyes, hair coloring agents, bleaching agents, UV absorbers, anti-UV agents, antibacterial agents, antifungal agents, melanin regulators, tanning accelerators, depigmenting agents, skin lightening agents, skin-coloring agents, liporegulators, weight-reduction agents, anti-acne agents, antiseborrhoeic agents, anti-ageing agents, anti-wrinkle agents, keratolytic agents, anti-inflammatory agents, anti-acne agents, antibiotics, anti-inflammatory agents, botanical extracts, imidazoles, refreshing agents, cicatrizing agents, vascular-protection agents, agents for the reduction of dandruff, seborrheic dermatitis, or psoriasis, shale oil and derivatives thereof, anti-psoriasis agents, corticosteroids depilating agents, agents for combating hair loss, reducing agents for permanent- waving, reflectants, essential oils and fragrances.

The pH of the composition of the invention is typically in the range of about 6.0 to about 10.0, and more typically in the range of about 6.0 to about 8.0.

Such post-foaming compositions are suitable for use as personal cleansing compositions, such as a body wash, facial wash or hand wash, for washing hair, or as shaving gels.

Also provided in accordance with the present invention is a method of manufacturing a personal cleansing composition comprising:
a) water;
b) one or more anionic surfactants in an amount of about 2 to about 20% by weight of the composition;
c) one or more amine oxide surfactants in an amount of about 1 to about 10% by weight of the composition;
d) a hydrophobically modified alkali-swellable acrylic emulsion (HASE) polymer in an amount of about 0.5 to about 3% polymer solids by weight of the composition;
e) a substantially water-insoluble oil in an amount of about 1% to about 50% by weight of the composition; and
f) a foaming agent comprising a non-polar saturated aliphatic hydrocarbon having from 3 to 6 carbon atoms;
the method comprising the steps of:
i) loading an amount of water into a suitable vessel;
ii) adding a HASE polymer, one or more anionic surfactants, one or more amine oxide surfactants and a substantially water-insoluble oil, sequentially in any order, with sufficient mechanical stirring to ensure that each material is fully dispersed before adding the next;
iii) continuing mixing until a uniform consistency is achieved; and
iv) adding a foaming agent comprising a non-polar saturated aliphatic hydrocarbon having from 3 to 6 carbon atoms.

Optionally, any of the surfactant or polymer constituents may be pre-mixed with water (with an equal amount of water being subtracted from the starting an amount of water added in step (i)) in any proportion before being added.

Optionally, one or more cationic conditioning agents may also be added into the water, before or after the addition of the HASE polymer and surfactants.

An exemplary, but non-limiting, method in accordance with the invention comprises the following steps:
i) dissolving one or more anionic surfactants in an amount of water;
ii) adding a HASE polymer emulsion, mixing until the polymer is substantially dispersed in the one or more anionic surfactants;
iii) adding a water-insoluble oil, mixing until the oil is substantially dispersed;
iv) adding one or more amine oxide surfactants; and
v) mixing until substantially uniform consistency is achieved.

Optionally, one or more cationic conditioning agents may also be added into the water, before the addition of the one or more anionic surfactants to the amount of water. Also, further optional minor ingredients, such as chelating agents, preservatives, fragrances, humectants may be added, typically after the addition of the HASE polymer, and further materials to be suspended if required (*e.g*. beads, scrubs, etc.), may also be added, typically after the addition of the water-insoluble oil.

The foaming agent is added last, after all of the other components have been mixed.

The formulation remains unstructured and generally of low viscosity until the addition of amine oxide, at which point the structure develops as the surfactants and polymer interact with each other. The final ingredient (hydrocarbon foaming agent) is added with appropriate mixing to ensure the final mixture is homogeneous, and the composition is filled into a suitable container.

Also provided in accordance with the present invention is a use of a composition comprising:
a) water;
b) one or more anionic surfactants in an amount of about 2 to about 20% by weight of the composition;
c) one or more amine oxide surfactants in an amount of about 1 to about 10% by weight of the composition;
d) a hydrophobically modified alkali-swellable acrylic emulsion (HASE) polymer in an amount of about 0.5 to about 3% polymer solids by weight of the composition;
e) a substantially water-insoluble oil in an amount of about 1% to about 50% by weight of the composition; and
f) a foaming agent comprising a non-polar saturated aliphatic hydrocarbon having from 3 to 6 carbon atoms,
as a personal cleansing composition, such as a body wash, facial wash or hand wash, for washing hair, or as a shaving gel.

Also provided in accordance with the present invention is a use of a composition comprising:
a) water;
b) one or more anionic surfactants in an amount of about 2 to about 20% by weight of the composition;
c) one or more amine oxide surfactants in an amount of about 1 to about 10% by weight of the composition;
d) a hydrophobically modified alkali-swellable acrylic emulsion (HASE) polymer in an amount of about 0.5 to about 3% polymer solids by weight of the composition;
e) a substantially water-insoluble oil in an amount of about 1% to about 50% by weight of the composition; and
f) a foaming agent comprising a non-polar saturated aliphatic hydrocarbon having from 3 to 6 carbon atoms
as a vehicle for delivery of one or more ingredients selected from emollients, moisturizers, conditioners, skin conditioners, hair conditioners, vitamins, pro-vitamins, antioxidants, free-radical scavengers, abrasives, dyes, hair coloring agents, bleaching agents, UV absorbers, anti-UV agents, antibacterial agents, antifungal agents, melanin regulators, tanning accelerators, depigmenting agents, skin lightening agents, skin-coloring agents, liporegulators, weight-reduction agents, anti-acne agents, antiseborrhoeic agents, anti-ageing agents, anti-wrinkle agents, keratolytic agents, anti-inflammatory agents, anti-acne agents, antibiotics, anti-inflammatory agents, botanical extracts, imidazoles, refreshing agents, cicatrizing agents, vascular-protection agents, agents for the reduction of dandruff, seborrheic dermatitis, or psoriasis, shale oil and derivatives thereof, anti-psoriasis agents, corticosteroids depilating agents, agents for combating hair loss, reducing agents for permanent- waving, reflectants, essential oils and fragrances to skin or hair.

The present invention will be further described with reference to the following examples, which are intended to be illustrative only, and in no way limiting upon the scope of the invention.

### EXAMPLES

**Table 1**

| | | **Formulations** | | | | | | |
|---|---|---|---|---|---|---|---|---|
| **Ingredients** | **Note** | **A** | **B** | **C** | **D** | **E** | **F** | **G** |
| Sodium Laureth Sulfate | 1 | 7.20 | 7.20 | 7.20 | | 6.30 | | |
| Sodium Lauroyl Sarcosinate | 2 | | | | | | | |
| Sodium Cocoyl Isethionate | 3 | | | | 6.75 | | | |
| Sodium Lauroyl Methyl Isethionate | 4 | | | | | | 2.70 | |
| Disodium Laureth Sulfosuccinate | 5 | | | | | | | 6.75 |
| Disodium Cocoyl Glutamate | 6 | | | | | | | |
| Lauramine Oxide | 7 | | | | | | 1.80 | |
| Lauramine/Myristamine Oxide | 8 | 1.80 | | 1.80 | 2.25 | 2.70 | | 2.25 |
| Cocamine Oxide | 9 | | 1.80 | | | | | |
| Lauric/Myristic Amidopropylamine Oxide | 10 | | | | | | | |
| Acrylates/Steareth-20 methacrylate crosspolymer | 11 | 0.90 | 0.90 | 0.90 | 0.90 | | 0.90 | 1.18 |
| Acrylates/Palmeth-25 acrylate crosspolymer | 12 | | | | | | | |
| Acrylates/Beheneth-25 methacrylate copolymer | 13 | | | | | 0.90 | | |
| Sodium Lithium Magnesium Silicate | 14 | | 0.18 | | 0.36 | | 0.36 | 0.36 |
| Polyquaternium-7 | 15 | 0.16 | | 0.16 | | 0.16 | | |
| PEG-200 Hydrogenated Glyceryl Palmate (and) PEG-7 Glyceryl Cocoate | 16 | | | | | | | |
| Tetrasodium Glutamate Diacetate | 17 | 0.09 | 0.09 | 0.09 | 0.09 | 0.09 | 0.09 | 0.09 |
| Glycerin | 18 | | 0.90 | 0.90 | | | | |
| DMDM Hydantoin | 19 | 0.18 | 0.18 | 0.18 | 0.18 | 0.18 | 0.18 | 0.18 |
| Fragrance Oil | 20 | 0.72 | 0.64 | 0.72 | 0.72 | 0.72 | 0.79 | 0.72 |
| Sunflower Oil | 21 | 5.00 | | | | | 3.00 | 2.00 |
| Rapeseed Oil | 21 | | 10.00 | | | | | |
| Paraffinum Liquidum | 22 | | | 3.00 | | | | |
| Jojoba Oil | 23 | | | | 2.00 | | | |
| Isopropyl Palmitate | 24 | | | | | 2.00 | | |
| Isopentane | 25 | 10.00 | 10.00 | 10.00 | 10.00 | 10.00 | 10.00 | 10.00 |

**Table 2**

| | | **Formulations** | | | | | | |
|---|---|---|---|---|---|---|---|---|
| **Ingredients** | **Note** | **H** | **I** | **J** | **K** | **L** | **M** | **N** |
| Sodium Laureth Sulfate | 1 | | | 5.00 | | 2.70 | 2.70 | 7.20 |
| Sodium Lauroyl Sarcosinate | 2 | | 6.75 | | | | | |
| Sodium Cocoyl Isethionate | 3 | | | | | | | |
| Sodium Lauroyl Methyl Isethionate | 4 | | | | 4.46 | | | |
| Disodium Laureth Sulfosuccinate | 5 | | | | | | | |
| Disodium Cocoyl Glutamate | 6 | 6.75 | | | | | | |
| Lauramine Oxide | 7 | | | | | | 1.80 | |
| Lauramine/Myristamine Oxide | 8 | 2.25 | 2.25 | 1.66 | 1.49 | 1.80 | | |
| Cocamine Oxide | 9 | | | | | | | |
| Lauric/Myristic Amidopropylamine Oxide | 10 | | | | | | | 1.80 |
| Acrylates/Steareth-20 methacrylate crosspolymer | 11 | 1.18 | | 1.00 | 1.00 | 0.60 | 0.52 | 1.50 |
| Acrylates/Palmeth-25 acrylate crosspolymer | 12 | | 1.18 | | | | | |
| Acrylates/Beheneth-25 methacrylate copolymer | 13 | | | | | | | |
| Sodium Lithium Magnesium Silicate | 14 | 0.36 | 0.36 | | | | | 0.18 |
| Polyquaternium-7 | 15 | | | | | 0.16 | 0.24 | 0.24 |
| PEG-200 Hydrogenated Glyceryl Palmate (and) PEG-7 Glyceryl Cocoate | 16 | | | | | 0.90 | | 0.90 |
| Tetrasodium Glutamate Diacetate | 17 | 0.09 | 0.09 | 0.10 | 0.09 | 0.09 | 0.09 | 0.09 |
| Glycerin | 18 | | | | | | 0.90 | 0.90 |
| DMDM Hydantoin | 19 | 0.18 | 0.18 | 0.18 | 0.18 | 0.18 | 0.18 | 0.18 |
| Fragrance Oil | 20 | 0.72 | 0.72 | 0.72 | 0.72 | 0.72 | 0.64 | 0.64 |
| Sunflower Oil | 21 | | | | | 25.00 | 50.00 | 8.00 |
| Rapeseed Oil | 21 | 5.00 | 10.00 | 5.00 | 5.00 | | | |
| Paraffinum Liquidum | 22 | | | | | | | |
| Jojoba Oil | 23 | | | | | | | |
| Isopropyl Palmitate | 24 | | | | | | | |
| Isopentane | 25 | 10.00 | 10.00 | 5.00 | 15.00 | 10.00 | 10.00 | 10.00 |

| | | | | | | | | |
|---|---|---|---|---|---|---|---|---|
| Notes 1 Steol CS 270 (Stepan Company) - supplied as 70% active paste 2 Surfacare L30 (Surfachem) - supplied as 30% active solution 3 Elfan AT84 (Akzo Nobel N.V.) - supplied as a powder 4 Iselux LQ-CLR-SB (Innospec) - supplied as a 35% active solution 5 Rewopol SB FA 30B (Evonik Industries A.G.) - supplied as 30% active solution 6 Plantapon ACG HC (BASF Personal Care) - supplied as 50% active solution 7 Mackamine LO E (Solvay Europe) - supplied as 30% active solution 8 Ammonyx LO (Stepan Company) - supplied as 30% active solution 9 Mackamine CS (Solvay Europe) - supplied as 30% active solution 10 Mackamine LMDO (Solvay Europe) - supplied as 30% active solution 11 Aculyn 88 (The Dow Chemical Company) - supplied as a 29% polymer solids dispersion 12 Synthalen W2000 (3V Sigma) - supplied as a 31% polymer solids dispersion 13 Novethix L-10 (The Lubrizol Corporation) - 30% polymer solids 14 Laponite XLS (BYK Additives) 15 Mackernium 007B (Solvay Europe) 16 Rewoderm LIS 80 (Evonik Industries A.G.) 17 Dissolvine GL38 (Akzo-Nobel N.V.) 18 Cremer Oleo 19 Microcare DH (Thor Personal Care) 20 Seven Scent 21 Food grade, acquired from retail store 22 Kemcare Ltd 23 IMCD N.V. 24 Tegosoft P (Evonik Industries A.G.) 25 Sigma Aldrich | | | | | | | | |

In each of the formulations listed in Tables 1 and 2, the balance was made up with water. All figures refer to % w/w as active substance with the exception of Rewoderm LIS 80 for which the inclusion levels are quoted as supplied.

All of the formulations A-N listed above have a pH in the range of 6.0 to 8.0, are opaque white, and have the consistency of self-supporting creams upon dispensation from a pressurised container. The application of gentle shearing forces (such as by rubbing onto a person's skin) causes a transformation into a dense foam or lather.

All of the formulations A-N listed above are able to act as personal cleansing compositions which incorporate insoluble benefit agents, *i.e.* the various oils, in a stable manner, have long shelf lives, are cost effective, and are easy to manufacture. The formulations are also able to incorporate other insoluble benefit agents in a stable manner if required.

Furthermore, the inventors have also found that the resulting compositions have advantageous sensory qualities when used for personal washing, in particular producing a voluminous yet creamy lather and a pleasant skin-feel during the washing process which persists as a conditioned feel after washing. The presence of significant amounts of oils in these formulations provides a number of benefits, namely a desirably creamy texture, deposition of oils onto the skin (countering the undesirable removal of natural lipids from the skin as experienced with most washing products) and a mildness benefit arising from a reduced capacity of the surfactants to interact with skin lipids.

It is of course to be understood that the present invention is not intended to be restricted to the foregoing example, which is described by way of illustration only.

## Claims

1. A post-foaming composition comprising:
a) water;
b) one or more anionic surfactants in an amount of about 2 to about 20% by weight of the composition;
c) one or more amine oxide surfactants in an amount of about 1 to about 10% by weight of the composition;
d) a hydrophobically modified alkali-swellable acrylic emulsion (HASE) polymer in an amount of about 0.5 to about 3% polymer solids by weight of the composition;
e) a water-insoluble oil from in an amount of about 1% to about 50% by weight of the total composition, wherein the solubility of the substantially water-insoluble oil in water at 20°C is less than 50% by weight of a water/oil mixture; and
f) a foaming agent comprising a non-polar saturated aliphatic hydrocarbon having from 3 to 6 carbon atoms.

2. A composition according to claim 1, wherein the HASE polymer is provided in the form of an aqueous liquid emulsion;
wherein the HASE polymer optionally comprises one or more selected from an acrylates/steareth-20 methacrylate copolymer, an acrylates/beheneth-25 methacrylate copolymer, an acrylates/steareth-20 methacrylate crosspolymer, an acrylates/palmeth-25 acrylate copolymer, and an acrylates/beheneth-25 methacrylate copolymer; and/or
wherein the HASE polymer is optionally present in an amount of about 1 to about 2% by weight of the composition.

3. A composition according to any preceding claim, wherein the one or more anionic surfactants comprise one or more selected from alkyl sulfates; ethoxylated alkyl sulfates; alpha olefin sulfonates; alkyl alkoxy carboxylates; acyl isethionates; acyl sarcosinates; sulfosuccinates; or acyl glutamates;
wherein the one or more anionic surfactants optionally comprise one or more selected from sodium lauryl sulfate, sodium cocosulfate, sodium laureth sulfate, sodium trideceth sulfate, sodium cocoyl isethionate, sodium cocoyl sarcosinate, disodium laureth sulfosuccinate, or sodium cocoyl glutamate;
wherein the one or more anionic surfactants optionally comprise sodium laureth sulfate; and/or
wherein the one or more anionic surfactants are optionally present in an amount of about 1 to about 10% by weight of the composition.

4. A composition according to any preceding claim, wherein the one or more amine oxide surfactants comprise alkamine oxides and alkylamidopropyl amine oxides containing at least one hydrocarbon chain containing at least eight carbon atoms.

5. A composition according to claim 4, wherein the one or more amine oxide surfactants comprise one or more selected from alkyl dimethyl amine oxides; or alkyl di(hydroxy lower alkyl)amine oxides; or alkamidopropyl dimethyl amine oxides,
wherein the alkyl groups are straight or branched chain, saturated or unsaturated and contain from 8 to about 22 carbon atoms and the lower alkyl groups contain from 1 to 7 carbon atoms;
wherein the alkyl dimethyl amine oxides optionally comprise one or more selected from lauryl dimethyl amine oxide, myristyl dimethyl amine oxide, dimethyl cocoamine oxide, myristyl/palmityl dimethyl amine oxide, myristyl/lauryl dimethyl amine oxide, cetyl dimethyl amine oxide, stearyl dimethyl amine oxide, and myristyl/cetyl dimethyl amine oxide;
the alkyl di(hydroxy lower alkyl)amine oxides optionally comprise one or more selected from bis(2-hydroxyethyl)cocoamine oxide, and bis(2-hydroxyethyl)stearylamine oxide; and
the alkamidopropyl dimethylamine oxides optionally comprise cocoamidopropyl dimethyl amine oxide.

6. A composition according to any of claims 4-5, wherein the one or more amine oxide surfactants comprise decyl dimethylamine oxide, lauryl dimethylamine oxide, stearyl dimethylamine oxide, oleyl dimethylamine oxide, coco dihydroxyethylamine oxide, cetyl N,N-dihydroxyethylamine oxide, oleyl N,N-dihydroxyethylamine oxide, cocamine oxide, cocamidopropylamine oxide, lauramidopropylamine oxide, oleamine oxide, oleamidopropylamine oxide, wheat germamidopropylamine oxide, isostearamido-propylamine oxide, stearamine oxide, stearamido-propylamine oxide, decylamine oxide, dihydroxyethyl C8-C18 alkoxypropylamine oxide, dihydroxyethyl stearamine oxide, myristamidopropylamine oxide, myristamine oxide, palmitamidopropylamine oxide, palmitamine oxide, undecylenamidopropylamine oxide, and mixtures of any two or more thereof;
wherein the one or more amine oxide surfactants optionally comprise lauramine oxide, myristamine oxide, cocamine oxide, lauramidopropyl amine oxide, myristamidopropyl amine oxide, cocamidopropyl amine oxide and mixtures of any two or more thereof.

7. A composition according to any preceding claim, wherein the one or more amine oxide surfactants are present in an amount of about 1 to about 5% by weight of the composition.

8. A composition according to any preceding claim, wherein the foaming agent comprises one or more compounds selected from iso-pentane, n-pentane, neo-pentane, iso-butane or n-butane;
wherein the foaming agent is optionally present in an amount of from about 2% to about 20% by weight of the composition.

9. A composition according to any preceding claim, wherein the oil is present in an amount of from about 2% to about 20% by weight of the composition.

10. A composition according to any preceding claim, wherein the pH of the invention is in the range of about 6.0 to about 8.0.

11. A composition according to any preceding claim, further comprising one or more ingredients selected from oils; natural or synthetic scrub particles; visual effect agents; humectants; cationic conditioning agents; preservatives, colorants, chelating agents, pH adjusters, or a clay-based rheology modifier; or
further comprising one or more ingredients selected from emollients, moisturizers, conditioners, skin conditioners, hair conditioners, vitamins, pro-vitamins, antioxidants, free-radical scavengers, abrasives, dyes, hair coloring agents, bleaching agents, UV absorbers, anti-UV agents, antibacterial agents, antifungal agents, melanin regulators, tanning accelerators, depigmenting agents, skin lightening agents, skin-coloring agents, liporegulators, weight-reduction agents, anti-acne agents, antiseborrhoeic agents, anti-ageing agents, anti-wrinkle agents, keratolytic agents, anti-inflammatory agents, anti-acne agents, antibiotics, anti-inflammatory agents, botanical extracts, imidazoles, refreshing agents, cicatrizing agents, vascular-protection agents, agents for the reduction of dandruff, seborrheic dermatitis, or psoriasis, shale oil and derivatives thereof, anti-psoriasis agents, corticosteroids depilating agents, agents for combating hair loss, reducing agents for permanent- waving, reflectants, essential oils and fragrances.

12. A method of manufacturing a personal cleansing composition according to any preceding claim, the method comprising the steps of:
i) loading an amount of water into a suitable vessel;
ii) adding a HASE polymer, one or more anionic surfactants, one or more amine oxide surfactants and a substantially water-insoluble oil, sequentially in any order, with sufficient mechanical stirring to ensure that each material is fully dispersed before adding the next;
iii) continuing mixing until a uniform consistency is achieved; and
iv) adding a foaming agent comprising a non-polar saturated aliphatic hydrocarbon having from 3 to 6 carbon atoms.

13. A method of manufacturing a personal cleansing composition according to claim 12, the method comprising the steps of:
i) dissolving one or more anionic surfactants in an amount of water;
ii) adding a HASE polymer emulsion, mixing until the polymer is substantially dispersed in the one or more anionic surfactants;
iii) adding a water-insoluble oil, mixing until the oil is substantially dispersed;
iv) adding one or more amine oxide surfactants; and
v) mixing until a uniform consistency is achieved; and
vi) adding a foaming agent comprising a non-polar saturated aliphatic hydrocarbon having from 3 to 6 carbon atoms.

14. Use of a composition according to any of claims 1-11 as a personal cleansing composition.

15. Use of a composition according to any of claims 1-11 as a vehicle for delivery of one or more ingredients selected from emollients, moisturizers, conditioners, skin conditioners, hair conditioners, vitamins, pro-vitamins, antioxidants, free-radical scavengers, abrasives, dyes, hair coloring agents, bleaching agents, UV absorbers, anti-UV agents, antibacterial agents, antifungal agents, melanin regulators, tanning accelerators, depigmenting agents, skin lightening agents, skin-coloring agents, liporegulators, weight-reduction agents, anti-acne agents, antiseborrhoeic agents, anti-ageing agents, anti-wrinkle agents, keratolytic agents, anti-inflammatory agents, anti-acne agents, antibiotics, anti-inflammatory agents, botanical extracts, imidazoles, refreshing agents, cicatrizing agents, vascular-protection agents, agents for the reduction of dandruff, seborrheic dermatitis, or psoriasis, shale oil and derivatives thereof, anti-psoriasis agents, corticosteroids depilating agents, agents for combating hair loss, reducing agents for permanent-waving, reflectants, essential oils and fragrances to skin or hair.

## Patentansprüche

1. Eine nachschäumende Zusammensetzung, umfassend:
a) Wasser;
b) ein oder mehrere anionische Tenside in einer Menge von etwa 2 bis etwa 20 Gew.-% der Zusammensetzung;
c) ein oder mehrere Aminoxid-Tenside in einer Menge von etwa 1 bis etwa 10 Gew.-% der Zusammensetzung;
d) ein hydrophob modifiziertes, alkalisch quellbares Acrylemulsionspolymer (HASE) in einer Menge von etwa 0,5 bis etwa 3 Gew.-% Polymerfeststoffen, bezogen auf die Zusammensetzung;
e) ein wasserunlösliches Öl in einer Menge von etwa 1 bis etwa 50 Gew.-% der Gesamtzusammensetzung, wobei die Löslichkeit des im Wesentlichen wasserunlöslichen Öls in Wasser bei 20 °C weniger als 50 Gew.-% einer Wasser/Öl-Mischung beträgt; und
f) ein Schaummittel, das einen unpolaren gesättigten aliphatischen Kohlenwasserstoff mit 3 bis 6 Kohlenstoffatomen umfasst.

2. Zusammensetzung nach Anspruch 1, wobei das HASE-Polymer in Form einer wässrigen flüssigen Emulsion bereitgestellt ist;
wobei das HASE-Polymer optional eines oder mehrere der folgenden umfasst: ein Acrylat/Steareth-20-Methacrylat-Copolymer, ein Acrylat/Beheneth-25-Methacrylat-Copolymer, ein Acrylat/Steareth-20-Methacrylat-Kreuzpolymer, ein Acrylat/Palmeth-25-Acrylat-Copolymer und ein Acrylat/Beheneth-25-Methacrylat-Copolymer umfasst; und/oder
wobei das HASE-Polymer optional in einer Menge von etwa 1 bis etwa 2 Gew.-% der Zusammensetzung vorhanden ist.

3. Zusammensetzung nach einem der vorstehenden Ansprüche, wobei das eine oder die mehreren anionischen Tenside eine oder mehrere der folgenden Substanzen umfassen: Alkylsulfate; ethoxylierte Alkylsulfate; Alpha-Olefinsulfonate; Alkylalkoxycarboxylate; Acylisethionate; Acylsarcosinate; Sulfosuccinate; oder Acylglutamate;
wobei das eine oder die mehreren anionischen Tenside optional eine oder mehrere der folgenden Substanzen umfassen: Natriumlaurylsulfat, Natriumcocosulfat, Natriumlaurethsulfat, Natriumtridecethsulfat, Natriumcocoylisethionat, Natriumcocoylsarcosinat, Dinatriumlaurethsulfosuccinat oder Natriumcocoylglutamat;
wobei das eine oder die mehreren anionischen Tenside optional Natriumlaurethsulfat umfassen; und/oder
wobei das eine oder die mehreren anionischen Tenside optional in einer Menge von etwa 1 bis etwa 10 Gew.-% der Zusammensetzung vorhanden sind.

4. Zusammensetzung nach einem der vorstehenden Ansprüche, wobei das eine oder die mehreren Aminoxid-Tenside Alkaminoxide und Alkylamidopropylaminoxide umfassen, die mindestens eine Kohlenwasserstoffkette mit mindestens acht Kohlenstoffatomen enthalten.

5. Zusammensetzung nach Anspruch 4, wobei das eine oder die mehreren Aminoxid-Tenside eine oder mehrere der folgenden Substanzen umfassen: Alkyl-dimethylaminoxide; oder Alkyl-di(hydroxy-niedrigalkyl)aminoxide; oder Alkylamidopropyl-dimethylaminoxide,
wobei die Alkylgruppen geradkettig oder verzweigt, gesättigt oder ungesättigt sind und 8 bis etwa 22 Kohlenstoffatome enthalten und die niederen Alkylgruppen 1 bis 7 Kohlenstoffatome enthalten;
wobei die Alkyl-dimethylaminoxide optional ein oder mehrere der folgenden enthalten: Lauryl-dimethylaminoxid, Myristyl-dimethylaminoxid, Dimethylcocoaminoxid, Myristyl/Palmityldimethylaminoxid, Myristyl/Lauryl-dimethylaminoxid, Cetyl-dimethylaminoxid, Stearyl-dimethylaminoxid und Myristyl/Cetyl-dimethylaminoxid;
die Alkyl-di(hydroxy-n-alkyl)aminoxide optional eines oder mehrere der folgenden umfassen: Bis(2-hydroxyethyl)cocoaminoxid und Bis(2-hydroxyethyl)stearylaminoxid; und
die Alkamidopropyl-dimethylaminoxide gegebenenfalls Cocoamidopropyldimethylaminoxid umfassen.

6. Zusammensetzung nach einem der Ansprüche 4 bis 5, wobei das eine oder die mehreren Aminoxid-Tenside Decyldimethylaminoxid, Lauryl-dimethylaminoxid, Stearyldimethylaminoxid, Oleyl-dimethylaminoxid, Coco-dihydroxyethylaminoxid, Cetyl-N,N-dihydroxyethylaminoxid, Oleyl-N,N-dihydroxyethylaminoxid, Cocaminoxid, Cocamidopropylaminoxid, Lauramidopropylaminoxid, Oleaminoxid, Oleamidopropylaminoxid, Weizenkeimamidopropylaminoxid, Isostearamidopropylaminoxid, Stearaminoxid, Stearamidopropylaminoxid, Decylaminoxid, Dihydroxyethyl-C8-C18-alkoxypropylaminoxid, Dihydroxyethylstearaminoxid, Myristamidopropylaminoxid, Myristaminoxid, Palmitamidopropylaminoxid, Palmitaminoxid, Undecylenamidopropylaminoxid und Mischungen aus zwei oder mehr davon enthalten;
wobei das eine oder die mehreren Aminoxid-Tenside optional Lauraminoxid, Myristaminoxid, Cocaminoxid, Lauramidopropylaminoxid, Myristamidopropylaminoxid, Cocamidopropylaminoxid und Mischungen aus zwei oder mehreren davon umfassen.

7. Zusammensetzung nach einem der vorstehenden Ansprüche, wobei das eine oder die mehreren Aminoxid-Tenside in einer Menge von etwa 1 bis etwa 5 Gew.-% der Zusammensetzung vorhanden sind.

8. Zusammensetzung nach einem der vorstehenden Ansprüche, wobei das Schaummittel eine oder mehrere Verbindungen umfasst, die aus Isopentan, n-Pentan, Neopentan, Isobutan oder n-Butan ausgewählt sind;
wobei der Schaumbildner optional in einer Menge von etwa 2 bis etwa 20 Gew.-% der Zusammensetzung vorhanden ist.

9. Zusammensetzung nach einem der vorstehenden Ansprüche, wobei das Öl in einer Menge von etwa 2 bis etwa 20 Gew.-% der Zusammensetzung vorhanden ist.

10. Zusammensetzung nach einem der vorstehenden Ansprüche, wobei der pH-Wert der Erfindung im Bereich von etwa 6,0 bis etwa 8,0 liegt.

11. Zusammensetzung nach einem der vorstehenden Ansprüche, die ferner einen oder mehrere Inhaltsstoffe umfasst, ausgewählt aus Ölen; natürlichen oder synthetischen Peelingpartikeln; Wirkstoffen für visuelle Effekte; Feuchthaltemitteln; kationischen Konditionierungsmitteln; Konservierungsmitteln, Farbstoffen, Chelatbildnern, pH-Reglern oder einem rheologischen Modifikator auf Tonbasis; oder
die ferner einen oder mehrere Inhaltsstoffe umfasst, ausgewählt aus Emollientien, Feuchthaltemitteln, Konditionierungsmitteln, Hautkonditionierungsmitteln, Haarkonditionierungsmitteln, Vitaminen, Provitaminen, Antioxidantien, Radikalfängern, Schleifmitteln, Farbstoffen, Haarfärbemitteln, Bleichmitteln, UV-Absorbern, UV-Schutzmitteln, antibakteriellen Mitteln, antimykotischen Mitteln, Melaninregulatoren, Bräunungsbeschleunigern, Depigmentierungsmitteln, Hautaufhellungsmitteln, Hautfärbemittel, Liporegulatoren, Gewichtsreduktionsmittel, Anti-Akne-Mittel, Antiseborrhoe-Mittel, Anti-Aging-Mittel, Anti-Falten-Mittel, Keratolytika, entzündungshemmende Mittel, Antibiotika, entzündungshemmende Mittel, Pflanzenextrakte, Imidazole, Erfrischungsmittel, Narbenmittel, Gefäßschutzmittel, Mittel zur Reduzierung von Schuppen, seborrhoische Dermatitis oder Psoriasis, Schieferöl und Derivate davon, Mittel gegen Psoriasis, Kortikosteroide, Enthaarungsmittel, Mittel gegen Haarausfall, Reduktionsmittel für Dauerwellen, Reflektoren, ätherische Öle und Duftstoffe.

12. Verfahren zur Herstellung einer Körperreinigungszusammensetzung gemäß einem der vorstehenden Ansprüche, wobei das Verfahren die folgenden Schritte umfasst:
i) Einfüllen einer Menge Wasser in einen geeigneten Behälter;
ii) Hinzufügen eines HASE-Polymers, eines oder mehrerer anionischer Tenside, eines oder mehrerer Aminoxid-Tenside und eines im Wesentlichen wasserunlöslichen Öls in beliebiger Reihenfolge unter ausreichendem mechanischem Rühren, um sicherzustellen, dass jedes Material vollständig dispergiert ist, bevor das nächste hinzugefügt wird;
iii) Fortsetzen des Mischens, bis eine gleichmäßige Konsistenz erreicht ist; und
iv) Zugabe eines Schaummittels, das einen unpolaren gesättigten aliphatischen Kohlenwasserstoff mit 3 bis 6 Kohlenstoffatomen enthält.

13. Verfahren zur Herstellung einer Körperreinigungszusammensetzung gemäß Anspruch 12, wobei das Verfahren die folgenden Schritte umfasst:
i) Auflösen eines oder mehrerer anionischer Tenside in einer Menge Wasser;
ii) Hinzufügen einer HASE-Polymeremulsion, Mischen, bis das Polymer im Wesentlichen in dem einen oder den mehreren anionischen Tensiden dispergiert ist;
iii) Hinzufügen eines wasserunlöslichen Öls, Mischen, bis das Öl im Wesentlichen dispergiert ist;
iv) Hinzufügen eines oder mehrerer Aminoxid-Tenside; und
v) Mischen, bis eine gleichmäßige Konsistenz erreicht ist; und
vi) Hinzufügen eines Schaummittels, das einen unpolaren gesättigten aliphatischen Kohlenwasserstoff mit 3 bis 6 Kohlenstoffatomen enthält.

14. Verwendung einer Zusammensetzung gemäß einem der Ansprüche 1 bis 11 als Körperreinigungsmittel.

15. Verwendung einer Zusammensetzung gemäß einem der Ansprüche 1 bis 11 als Träger für die Abgabe eines oder mehrerer Inhaltsstoffe, ausgewählt aus Emollientien, Feuchthaltemitteln, Konditionierungsmitteln, Hautkonditionierungsmitteln, Haarkonditionierungsmitteln, Vitaminen, Provitaminen, Antioxidantien, Radikalfängern, Abrasivmitteln, Farbstoffen, Haarfärbemitteln, Bleichmitteln, UV-Absorbern, UV-Schutzmitteln, antibakteriellen Mitteln, antimykotischen Mitteln, Melaninregulatoren, Bräunungsbeschleuniger, Depigmentierungsmittel, Hautaufheller, Hautfärbemittel, Liporegulatoren, Gewichtsreduktionsmittel, Anti-Akne-Mittel, Antiseborrhoe-Mittel, Anti-Aging-Mittel, Anti-Falten-Mittel, Keratolytika, entzündungshemmende Mittel, Antibiotika, entzündungshemmende Mittel, Pflanzenextrakte, Imidazole, Erfrischungsmittel, Narbenheilungsmittel, Gefäßschutzmittel, Mittel zur Reduzierung von Schuppen, seborrhoischer Dermatitis oder Psoriasis, Schieferöl und Derivate davon, Mittel gegen Psoriasis, Kortikosteroide, Enthaarungsmittel, Mittel gegen Haarausfall, Reduktionsmittel für Dauerwellen, Reflektoren, ätherische Öle und Duftstoffe für Haut oder Haare.

## Revendications

1. Composition post-moussage comprenant :
a) de l'eau ;
b) un ou plusieurs tensioactifs anioniques en une quantité d'environ 2 à environ 20 % en poids de la composition ;
c) un ou plusieurs tensioactifs d'oxyde d'amine en une quantité d'environ 1 à environ 10 % en poids de la composition ;
d) un polymère d'émulsion acrylique gonflable aux alcalis hydrophobiquement modifié (HASE) en une quantité d'environ 0,5 à environ 3 % de solides polymères en poids de la composition ;
e) une huile insoluble dans l'eau en une quantité d'environ 1 % à environ 50 % en poids de la composition totale, dans laquelle la solubilité dans l'eau de l'huile sensiblement insoluble dans l'eau à 20 °C est inférieure à 50 % en poids d'un mélange eau/huile ; et
f) un agent moussant comprenant un hydrocarbure aliphatique saturé non polaire présentant de 3 à 6 atomes de carbone.

2. Composition selon la revendication 1, dans laquelle le polymère HASE est fourni sous la forme d'une émulsion liquide aqueuse ;
dans laquelle le polymère HASE comprend facultativement un ou plusieurs éléments sélectionnés parmi un copolymère acrylates/stéareth-20 méthacrylate, un copolymère acrylates/béhéneth-25 méthacrylate, un polymère réticulé acrylates/stéareth-20 méthacrylate, un copolymère acrylates/palmeth-25 acrylate, et un copolymère acrylates/béhéneth-25 méthacrylate ; et/ou
dans laquelle le polymère HASE est facultativement présent en une quantité d'environ 1 à environ 2 % en poids de la composition.

3. Composition selon une quelconque revendication précédente, dans laquelle les un ou plusieurs tensioactifs anioniques comprennent un ou plusieurs éléments sélectionnés parmi les sulfates d'alkyle ; les sulfates d'alkyle éthoxylés ; les sulfonates d'alpha-oléfine ; les carboxylates d'alkylalcoxy ; les iséthionates d'acyle ; les sarcosinates d'acyle ; les sulfosuccinates ; ou les glutamates d'acyle ;
dans laquelle les un ou plusieurs tensioactifs anioniques comprennent facultativement un ou plusieurs éléments sélectionnés parmi laurylsulfate de sodium, cocosulfate de sodium, laurethsulfate de sodium, tridécethsulfate de sodium, cocoyliséthionate de sodium, cocoylsarcosinate de sodium, laurethsulfosuccinate disodique, ou cocoylglutamate de sodium ;
dans laquelle les un ou plusieurs tensioactifs anioniques comprennent facultativement du laurethsulfate de sodium ; et/ou
dans laquelle les un ou plusieurs tensioactifs anioniques sont facultativement présents en une quantité d'environ 1 à environ 10 % en poids de la composition.

4. Composition selon une quelconque revendication précédente, dans laquelle les un ou plusieurs tensioactifs d'oxyde d'amine comprennent des oxydes d'alkamine et des oxydes d'amine d'alkylamidopropyle contenant au moins une chaîne d'hydrocarbures contenant au moins huit atomes de carbone.

5. Composition selon la revendication 4, dans laquelle les un ou plusieurs tensioactifs d'oxyde d'amine comprennent un ou plusieurs éléments sélectionnés parmi les oxydes d'amine diméthylique d'alkyle ; ou les oxydes d'alkyle di(alkyl inférieur d'hydroxy)amine ; ou les oxydes d'amine diméthylique d'alkamidopropyle,
dans laquelle les groupes alkyle sont à chaîne droite ou ramifiée, saturés ou insaturés et contiennent d'environ 8 à environ 22 atomes de carbone et les groupes alkyle inférieur contiennent d'environ 1 à 7 atomes de carbone ;
dans laquelle les oxydes d'amine diméthylique d'alkyle comprennent facultativement un ou plusieurs éléments sélectionnés parmi l'oxyde d'amine diméthylique de lauryle, l'oxyde d'amine diméthylique de myristyle, l'oxyde de cocoamine diméthylique, l'oxyde d'amine diméthylique de myristyle/palmityle, l'oxyde d'amine diméthylique de myristyle/lauryle, l'oxyde d'amine diméthylique de cétyle, l'oxyde d'amine diméthylique de stéaryle et l'oxyde d'amine diméthylique de myristyle/cétyle ;
dans laquelle les oxydes d'alkyle di(alkyl inférieur d'hydroxy)amine comprennent facultativement un ou plusieurs éléments sélectionnés parmi oxyde de bis(2-hydroxyéthyl)cocoamine, et oxyde de bis(2-hydroxyéthyl) stéarylamine ; et
les oxydes d'amine diméthylique d'alkamidopropyle comprennent facultativement de l'oxyde d'amine diméthylique de cocoamidopropyle.

6. Composition selon l'une quelconque des revendications 4-5, dans laquelle les un ou plusieurs tensioactifs d'oxyde d'amine comprennent l'oxyde de diméthylamine de décyle, l'oxyde de diméthylamine de lauryle, l'oxyde de diméthylamine de stéaryle, l'oxyde de diméthylamine d'oléyle, l'oxyde de dihydroxyéthylamine de coco, l'oxyde de N,N-dihydroxyéthylamine de cétyle, l'oxyde de N,N-dihydroxyéthylamine d'oléyle, l'oxyde de cocamine, l'oxyde de cocamidopropylamine, l'oxyde de lauramidopropylamine, l'oxyde d'oléamine, l'oxyde d'oléamidopropylamine, l'oxyde de germamidopropylamine de blé, l'oxyde d'isostéaramido-propylamine, l'oxyde de stéaramine, l'oxyde de stéaramido-propylamine, l'oxyde de décylamine, l'oxyde d'alcoxypropylamineC8-C18 de dihydroxyéthyle, l'oxyde de stéaramine de dihydroxyéthyle, l'oxyde de myristamidopropylamine, l'oxyde de myristamine, l'oxyde de palmitamidopropylamine, l'oxyde de palmitamine, l'oxyde d'undécyleamidopropylamine, et des mélange de deux quelconques ou plus de ceux-ci ;
dans laquelle les un ou plusieurs tensioactifs d'oxyde d'amine comprennent facultativement l'oxyde de lauramine, l'oxyde de myristamine, l'oxyde de cocamine, l'oxyde d'amine de laramidopropyle, l'oxyde d'amine de myristamidopropyle, l'oxyde d'amine de cocamidopropyle et des mélanges de deux quelconques ou plus de ceux-ci.

7. Composition selon une quelconque revendication précédente, dans laquelle les un ou plusieurs tensioactifs d'oxyde d'amine sont présents en une quantité d'environ 1 à environ 5 % en poids de la composition.

8. Composition selon une quelconque revendication précédente, dans laquelle l'agent moussant comprend un ou plusieurs composés sélectionnés parmi l'iso-pentane, le n-pentane, le néo-pentane, l'iso-butane ou le n-butane ;
dans laquelle l'agent moussant est facultativement présent en une quantité d'environ 2 % à environ 20 % en poids de la composition.

9. Composition selon une quelconque revendication précédente, dans laquelle l'huile est présente en une quantité d'environ 2 % à environ 20 % en poids de la composition.

10. Composition selon une quelconque revendication précédente, dans laquelle le pH de l'invention est dans la plage d'environ 6,0 à environ 8,0.

11. Composition selon une quelconque revendication précédente, comprenant en outre un ou plusieurs ingrédients sélectionnés parmi des huiles ; des particules exfoliantes naturelles ou synthétiques ; des agents d'effet visuel ; des agents humidifiants ; des agents de revitalisation cationiques ; des conservateurs, des colorants, des agents de chélation, des correcteurs de pH, ou un modificateur de rhéologie à base d'argile ; ou
comprenant en outre un ou plusieurs ingrédients sélectionnés parmi des émollients, des produits hydratants, des revitalisants, des revitalisants cutanés, des revitalisants pour cheveux, des vitamines, des pro-vitamines, des anti-oxydants, des piégeurs de radicaux libres, des abrasifs, des teintures, des agents de coloration des cheveux, des agents blanchissants, des absorbeurs d'UV, des agents anti-UV, des agents antibactériens, des agents antifongiques, des régulateurs de mélanine, des accélérateurs de bronzage, des agents de dépigmentation, des agents d'éclaircissement de la peau, des agents de coloration de la peau, des liporégulateurs, des agents de réduction de poids, des agents anti-acné, des agents antiséborrhéiques, des agents antivieillissement, des agents anti-rides, des agents kératolytiques, des agents anti-inflammatoires, des agents anti-acné, des antibiotiques, des agents anti-inflammatoires, des extraits botaniques, des imidazoles, des agents rafraîchissants, des agents de cicatrisation, des agents de protection vasculaire, des agents pour la réduction des pellicules, de la dermatite séborrhéique ou le psoriasis, l'huile de schiste et des dérivés de celle-ci, des agents anti-psoriasis, des corticostéroïdes, des agents dépilatoires, des agents pour lutter contre la perte des cheveux, des agents de réduction pour l'ondulation permanente, les agents réfléchissants, les huiles essentielles et les parfums.

12. Procédé de fabrication d'une composition de nettoyage personnel selon une quelconque revendication précédente, le procédé comprenant les étapes consistant à ;
i) charger une quantité d'eau dans un récipient approprié ;
ii) ajouter un polymère HASE, un ou plusieurs tensioactifs anioniques, un ou plusieurs tensioactifs d'oxyde d'amine et une huile sensiblement insoluble dans l'eau, de manière séquentielle dans tout ordre, avec une agitation mécanique suffisante pour s'assurer que chaque matériau soit entièrement dispersé avant d'ajouter le suivant ;
iii) continuer à mélanger jusqu'à ce qu'une consistance homogène soit obtenue ; et
iv) ajouter un agent moussant comprenant un hydrocarbure aliphatique saturé non polaire présentant de 3 à 6 atomes de carbone.

13. Procédé de fabrication d'une composition de nettoyage personnel selon la revendication 12, le procédé comprenant les étapes consistant à :
i) dissoudre un ou plusieurs tensioactifs anioniques dans une quantité d'eau ;
ii) ajouter une émulsion de polymère HASE, mélanger jusqu'à ce que le polymère soit sensiblement dispersé dans les un ou plusieurs tensioactifs anioniques ;
iii) ajouter une huile insoluble dans l'eau, mélanger jusqu'à ce que l'huile soit sensiblement dispersée ;
iv) ajouter un ou plusieurs tensioactifs d'oxyde d'amine ; et
v) mélanger jusqu'à ce qu'une consistance homogène soit obtenue ; et
vi) ajouter un agent moussant comprenant un hydrocarbure aliphatique saturé non polaire présentant de 3 à 6 atomes de carbone.

14. Utilisation d'une composition selon l'une quelconque des revendications 1 à 11 en tant que composition de nettoyage personnel.

15. Utilisation d'une composition selon l'une quelconque des revendications 1 à 11 en tant que vecteur pour l''administration d'un ou plusieurs ingrédients sélectionnés parmi des émollients, des produits hydratants, des revitalisants, des revitalisants cutanés, des revitalisants pour cheveux, des vitamines, des pro-vitamines, des anti-oxydants, des piégeurs de radicaux libres, des abrasifs, des teintures, des agents de coloration des cheveux, des agents blanchissants, des absorbeurs d'UV, des agents anti-UV, des agents antibactériens, des agents antifongiques, des régulateurs de mélanine, des accélérateurs de bronzage, des agents de dépigmentation, des agents d'éclaircissement de la peau, des agents de coloration de la peau, des liporégulateurs, des agents de réduction de poids, des agents anti-acné, des agents antiséborrhéiques, des agents antivieillissement, des agents anti-rides, des agents kératolytiques, des agents anti-inflammatoires, des agents anti-acné, des antibiotiques, des agents anti-inflammatoires, des extraits botaniques, des imidazoles, des agents rafraîchissants, des agents de cicatrisation, des agents de protection vasculaire, des agents pour la réduction des pellicules, de la dermatite séborrhéique ou le psoriasis, l'huile de schiste et des dérivés de celle-ci, des agents anti-psoriasis, des corticostéroïdes, des agents dépilatoires, des agents pour lutter contre la perte des cheveux, des agents de réduction pour l'ondulation permanente, les agents réfléchissants, les huiles essentielles et les parfums.
